# FASCICULE DE BREVET EUROPEEN

(11) **EP 1 147 765 B1**
(45) Date de publication et mention de la délivrance du brevet: **03.05.2006**
(21) Numéro de dépôt: 01400825.4
(22) Date de dépôt: 30.03.2001
(51) Int. Cl.: A61K 8/37, A61K 8/46, A61K 8/55, A61K 8/60, A61K 8/44, A61Q 5/00

(54) **Nouvel actif, composition le renfermant et utilisation en cosmétique, dermocosmétique, dermopharmacie ou pharmacie ou sur des supports tisses ou non tisses**
Neuer Actif, sowie ihre Zusammensetzungen , ihre Verwendung in der Kosmetik, Dermokosmetik , Dermopharmacy, Pharmacy und auf Vliesstoffe
New active, compositions containing it and it's use in cosmetic, dermocosmetic, dermopharmacy or pharmacy, on woven or nonwoven fabrics

(30) Priorité: 06.04.2000 FR 0004412
(43) Date de publication de la demande: 24.10.2001
(73) Titulaire: SOCIETE D'EXPLOITATION DE PRODUITS POUR LES INDUSTRIES CHIMIQUES, S.E.P.P.I.C., 75321 Paris Cédex 07 (FR)
(72) Inventeur: Stoltz, Corinne, 94320 Thiais (FR); Geoffroy, Hervé, 77150 Lesigny (FR)
(74) Mandataire: Conan, Philippe Claude

(56) Documents cités:
- EP-A- 1 064 912
- WO-A-00/44337
- WO-A-98/18446
- WO-A-99/08649
- DE-A- 4 109 976
- US-A- 6 071 541

## Description

La présente invention a pour objet une nouvelle composition cosmétique de composés à structure lipoaminoacide, à activité apaisante.

Dans la vie moderne, les cheveux sont agressés par divers facteurs externes au corps humain : il s'agit notamment de la pollution atmosphérique, des rayonnements ultraviolets, qu'ils soient naturels ou artificiels ou d'autres stress tels que les stress mécaniques ou chimiques.

Du fait de la plus en plus grande prise en compte de ces problèmes, notamment dans les lieux fortement urbanisés, l'aspect protection du cheveu est devenu prépondérant dans la recherche de nouveaux produits cosmétiques.

La demanderesse a développé le nouveau concept d'actif capillaire multiprotecteur et thermoactif, pour cheveux stressés, en réponse aux agressions ou aux sensations d'agression ressenties par le sujet et, plus particulièrement, en réponse aux méfaits d'origine radicalaire. Elle a découvert que le stress du système capillaire induit la formation de radicaux libres et de peroxydes lipidiques. A l'issue d'une cascade de réactions radicalaires (réaction de Fenton) qui altère le métabolisme protéique, la division cellulaire est ralentie et la fibre capillaire est endommagée au niveau de la kératine du cheveu et de sa racine. Le cheveu devient alors fragile, écaillé et terne. Pour une protection efficace de la totalité du cheveu, il est donc primordial, pour un actif capillaire, de pouvoir réduire le taux de peroxydes lipidiques formés à la surface des cheveux.

En raison de leur structure amphiphile, Les composés à structure lipoaminoacide, comme que ceux décrits dans les demandes internationales de brevet publiées sous les numéros WO92/20647, WO92/21318, WO94/26694 et WO94/27561, sont des vecteurs biologiques particulièrement intéressants, car ils régulent la physiologie cutanée. Ils s'avèrent donc appropriés à de multiples applications, notamment en cosmétique.

La demande de brevet européen EP 1064 912, citée comme état de la technique au sens de l'article 54(3) CBE, décrit des compositions cosmétiques incluant du panthénol et des dérivés acylés d'hydrolysats de protéines.

Le panthénol ou 2,4-dihydroxy N-(3-hydroxy propyl) 3,3-diméthyl butanamide de formule :

HO-(CH₂)₃NH-C(=O)-CH(OH)-C(CH₃)₂-CH₂-OH,

est un composé couramment utilisé dans les traitements du cheveu et du scalp.

Or, la demanderesse a trouvé que les compositions comprenant comme principes actifs, l'association de lipoaminoacides avec des dérivés de panthénol possédaient à la fois une activité antiradicalaire, une activité stimulatrice de la division cellulaire, exacerbée par la chaleur et une activité protectrice de la kératine empêchant la formation d'écailles. La demanderesse a aussi trouvé que cette activité était le résultat d'une synergie issue de l'association de ces deux familles de principes actifs.

L'invention a pour objet une composition caractérisée en ce qu'elle comprend à titre de principe actif, au moins un composé de formule (I): 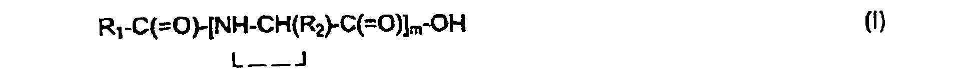 ou ses sels, dans laquelle R₁ représente la chaîne caractérisante d'un acide gras, saturé ou insaturé, linéaire ou ramifié, comportant de 8 à 30 atomes de carbone, R₂ représente la chaîne caractérisante d'un acide aminé et m est compris entre 1 et 5, et au moins un composé de formule (II) :

ZO-(CH₂)₃-NH-C(=O)-CH(OH)-C(CH₃)₂-CH₂-OH (II)

dans laquelle Z. représente un radical dérivé du phosphore de formule : P(R₃O)(OM)(=O)-, dans lequel OM représente un radical OH libre ou salifié, ou bien sous forme de sel alcalin comme le sel de sodium, ou le sel de potassium, ou bien sous forme de sel d'ammonium ou bien sous forme de sel d'un amino-alcool comme de sel de (2-hydroxy éthyl) ammonium et R₃ un radical dérivé de polysiloxanes alkoxyles.

Le composé de formule (I) présent dans la composition objet de la présente invention, peut être sous forme d'acide libre ou sous forme partiellement ou totalement salifiée. Lorsque le composé de formule (1) est sous forme salfiée, Il s'agit noment salifiée. Lorsque le composé de formule (I) est sous forme salifiée, II s'agit notamment de sels alcalins tels que les sels de sodium, de potassium ou de lithium, de sels alcalino-terreux tels que les sels de calcium, de magnésium ou de strontium ; de sel d'ammonium ou de sel d'un amino-alcool comme le sel de (2-hydroxy éthyl) ammonium. Il peut aussi s'agir de sels métalliques tels que les sels divalents de zinc ou de manganèse, les sels trivalents de fer, de lanthane, de cérium ou d'aluminium.

L'expression "chaîne caractérisante" utilisée pour définir les radicaux R₁ et R₂, désigne la chaîne principale non fonctionnelle de l'acide gras ou de l'acide aminé considéré.

Ainsi, pour un acide gras répondant à la formule générale R₁-C(=O)-OH, la chaîne caractérisante sera la chaîne représentée par R₁. Le radical R₁ représente notamment un radical comportant de 8 à 22 atomes de carbone choisi parmi les radicaux octyle, nonyle, décyle, undécyle, dodécyle, tridécyle, tétradécyle, pentadécyle, hexadécyle, heptadécyle, octadécyle, nonadécyle, eicosyle, uneicosyle, docosyle, heptadécènyle, eicosènyle, uneicosènyle, docosènyle ou heptadécadiènyle ou décènyle.

Selon un premier aspect particulier, l'invention a pour objet une composition telle que décrite précédemment pour laquelle, dans la formule (I), le groupe R₁-C(=O)- comporte de 8 à 22 atomes de carbone et représente notamment l'un des radicaux octanoyle (caprylyle), décanoyle, undécylènoyle, dodécanoyle (lauroyle), tétradécanoyle (myristyle), hexadécanoyle (palmitoyle), octadécanoyle (stéaryle), eicosanoyle (arachidoyle), docosanoyle (béhènoyle), 8-octadécènoyle (oléyle), éicosinoyte (gadoloyle), 13-docosènoyle (érucyle), 9,12-octadécadiènoyle (linoléoyle) ou 9,12,15-octadécatriénoyle (linolénoyle).

Selon une première variante préférée de la présente invention, dans la formule (I), le fragment R₁-C(=O) comporte de 12 à 18 atomes de carbone.

Pour un acide aminé représenté par la formule générale (IIIa):

H_{z}N-CH(R₂)-C(=O)-OH (IIIa),

comme pour un acide aminé cyclique représenté par la formule (Illb) : 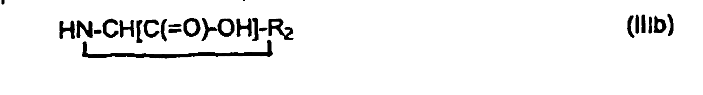 la chaîne caractérisante sera la chaîne représentée par R₂.

R₂ représente notamment la chaîne caractérisante d'un des acides aminés choisis parmi la glycine, l'alanine, la sérine, l'acide aspartique, l'acide glutamique, la valine, la thréonine, l'arginine, la lysine, la proline, la leucine, la phénylalanine, l'isoleucine, l'histidine, la tyrosine, le tryptophane, l'asparagine, la cystéine, la cystine, la méthionine, l'hydroxy proline, l'hydroxy lysine ou l'ornithine.

Selon un deuxième aspect particulier, l'invention a pour objet une composition telle que définie précédemment, comprenant au moins un composé de formule (I) choisi parmi les dérivés N-acylés de l'acide glutamique de l'acide aspartique de l'alanine ou de la glycine.

Dans la formule (I) telle que définie précédemment, m est un nombre décimal inférieur ou égal à 2, plus particulièrement inférieur ou égal à 1,4 ; m est plus particulièrement égal à 1.

Selon un troisième aspect particulier, l'invention a pour objet une composition telle que décrite précédemment, comprenant un seul composé de la formule (I).

Selon un quatrième aspect particulier, l'invention a pour objet une composition telle que décrite précédemment, comprenant un mélange de composés de la formules (I), et notamment, ou bien un mélange de composés de formules (I) comportant toutes le même fragment R₁-C(=O), ou bien un mélange de composés de formules (I) dans lesquelles m est égal à 1 et comportant toutes le même fragment

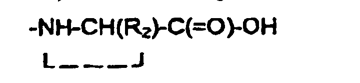

Les composés de formules (I) sont généralement obtenus par N-acylation de composés de formules (IIIa) ou (IIIb), telles que définies précédemment ou de leurs sels. Lorsqu'il s'agit d'un mélange de composés de formules (I), il est par exemple obtenu par N-acylation du mélange d'acides aminés résultant de l'hydrolyse totale ou partielle de protéines de toutes origines. Ces protéines peuvent être d'origine animale, telles que, par exemple, le collagène, l'élastine, la protéine de chair de poisson, la gélatine de poissons, la kératine ou la caséine, d'origine végétale, comme les protéines de céréales, de fleurs ou de fruits, telles que par exemple, les protéines issues du soja, du tournesol, de l'avoine, du blé, du maïs, de l'orge, de la pomme de terre, du lupin, de la féverole, de l'amande douce, du kiwi, de la mangue ou de la pomme ; il peut s'agir aussi de protéines obtenues à partir de chorelles (algues unicellulaires), d'algues roses, de levures ou de la soie. Cette hydrolyse est réalisée par exemple, par chauffage à des températures comprises entre 60 et 130°C d'une protéine placée dans un milieu acide ou alcalin. Cette hydrolyse peut également être réalisée par voie enzymatique avec une protéase, couplée éventuellement à une post-hydrolyse alcaline ou acide. Quand m est supérieur à 1, R₂ représente une seule même chaîne ou bien plusieurs chaînes caractérisant différents acides aminés, selon la protéine hydrolysée et le degré d'hydrolyse. Les aminogrammes de quelques protéines d'origine végétales sont consignées dans le tableau suivant :

**Tableau A**

| | Origine de la protéine (proportions en acides aminés exprimées en % pondéraux) | | | |
|---|---|---|---|---|
| | Avoine | Soja | Blé | Tournesol |
| Glycine | 6,9 | 4,2 | 3,2 | 6.2 |
| Alanine | 5,9 | 4,2 | 2,6 | 4,8 |
| Serine | 5,6 | 5,1 | 1,7 | 5,1 |
| Acide aspartique | 16,2 | 11,7 | 3,4 | 10,6 |
| Acide glutamique | 28,3 | 19,1 | 37,9 | 23,6 |
| Valine | 2,9 | 5.0 | 4,2 | 4,8 |
| Thréonine | 3,1 | 3,9 | 2,7 | 4,4 |
| Arginine | 6,6 | 7,8 | 3,7 | 8,4 |
| Lysine | 3,6 | 6,2 | 1,9 | 3,2 |
| Proline | 4,7 | 5,4 | 11,7 | 3,0 |
| Leucine | 6,4 | 8,1 | 7,1 | 6,4 |
| Phénylalanine | 1,4 | 5,0 | 5,4 | 4,3 |
| Isoleucine | 2,2 | 4,8 | 3,7 | 4,1 |
| Histidine | 1,7 | 2,6 | 2,4 | 2,0 |
| Tyrosine | 1,5 | 3,5 | 3.1 | 2,7 |
| Méthionine | 1,2 | 1,2 | 1,6 | 1,8 |
| Cystéine 1 Cystine | 1,9 | 1,5 | 1.9 | 1.9 |
| Tryptophane | - | 1,0 | 1,0 | 1,3 |

**Tableau A (suite)**

| | Origine de la protéine (proportions en acides aminés exprimées en % pondéraux) | | | |
|---|---|---|---|---|
| | Lupin | Pomme de terre | Féverole | Maïs |
| Glycine | 0,9 | 4,8 | 4,0 | 2,4 |
| Alanine | 2,4 | 5,0 | 4,0 | 7,95 |
| Serine | 6,1 | 5,8 | 4,9 | 5,1 |
| Acide aspartique | 15,8 | 12,5 | 10,5 | 10,6 |
| Acide glutamique | 8,0 | 11,5 | 16,8 | 23,6 |
| Valine | 7,9 | 7,1 | 4,5 | 4,8 |
| Thréonine | 8,1 | 6,1 | 3,6 | 4,4 |
| Arginine | 16,1 | 5,0 | 9,21 | 8,4 |
| Lysine | 7.1 | 7,8 | 6,5 | 6,2 |
| Proline | - | 5,1 | 4,4 | 3,0 |
| Leucine | 7,45 | 10,4 | 7,4 | 8,1 |
| Phénylalanine | 8,6 | 6,4 | 4,4 | 4,3 |
| Isoleucine | 8,7 | 6,1 | 3,9 | 4,1 |
| Histidine | - | 2,2 | 2,6 | 2,0 |
| Tyrosine | - | 5,7 | 3,6 | 2,7 |
| Méthionine | 0,6 | 2,4 | 0,8 | 1,8 |
| Cystéine/Cystine | - | 1,6 | 1,7 | 1,9 |
| Tryptophane | 1,2 | 1,4 | 1,2 | 1,3 |
| Omithine | 0,4 | - | - | - |

La réaction d'acylation est connue de l'homme du métier. Elle est décrite par exemple dans la demande internationale publiée sous le numéro WO 98/09611. Elle est mise en oeuvre indifféremment sur un acide aminé ou sur un mélange d'acides aminés. L'agent d'acylation consiste généralement en un dérivé activé d'un acide carboxylique de formule R₁C(=O)-OH. tel qu'un un anhydride symétrique de cet acide ou un halogénure d'acide comme le chlorure d'acide ou le bromure d'acide. Il peut aussi consister en un mélange de dérivés activés d'acides carboxyliques issus d'huiles ou graisses naturelles d'origine animales ou végétales telles que les huiles de coprah, de palmiste, de palme, de soja, de colza, de maïs, le suif de boeuf, l'huile spermaceti ou l'huile de hareng.

L'invention a tout particulièrement pour objet une composition telle que définie précédemment, pour laquelle le composé de formule (I) est un N-lauroyl aminoacide ou un mélange de N-cocoyl aminoacides. Comme exemple de tels mélanges, il y a le PROTEOL™ SAV 50S ou le PROTEOL™ OAT commercialisés par la société SEPPIC.

Les composés de formule (II) telle que définie précédemment, sont préparés par réaction de polysiloxanes alkoxylés et phosphatés, tels que ceux dont la préparation est décrite dans les brevets américain publiés sous les numéros US 5,070,171, US 5,091493, US 5,093,452, US 5,100956, US 5,149765 ou US 5,243,028, avec le panthénol. Un de ces composés est disponible dans le commerce sous le nom de PECOSIL™ SPP 50, appelé suivant la dénomination INCI : Potassium dimethicone copolyol panthènyl phosphate.

Des agents tensioactifs à groupements phosphate de la famille des polysiloxanes alkoxylés et phosphatés sont décrits dans les brevets américain publiés sous les numéros US 5,070,171, US 5,091,493, US 5,093,452, US 5,100,956, US 5,149,765 ou US 5,243, 028 et plus particulièrement un des sels de diméthicone copolyol phosphate commercialisés sous les noms de PECOSIL™ PS-100, PECOSIL™ PS-200 ou PECOSIL™ WDS-100.

Selon un cinquième aspect particulier, invention a pour objet une composition telle que définie précédemment, dans laquelle le composé de formule (II) est le potassium diméthicone copolyol panthényl phosphate ou PECOSIL™ SPP-50. Selon cette variante, la composition peut aussi comprendre un ou plusieurs agents tensioactifs à groupements phosphate de la famille des polysiloxanes alkoxylés et phosphatés tels que ceux décrits dans les brevets américain publiés sous les numéros US 5,070,171, US 5,091493, US 5,093,452, US 5,100,956, US 5,149765 ou US 5,243, 028 et plus particulièrement un des sels de diméthicone copolyol phosphate commercialisés sous les noms de PECOSIL™ PS 100, PECOSIL™ PS-200 ou PECOSIL™ WDS-100.

La composition, objet de la présente invention est préparée par des méthodes connues de l'homme du métier. Outre les principes actifs, la composition selon l'invention comprend des véhicules minéraux ou organiques couramment utilisés dans la fabrication de compositions destinées à être formulées en préparations à usage cosmétique et/ou pharmaceutique. On peut citer par exemple l'eau ou les mélanges eau-alcohol, tels que les solutions aqueuses d'éthanol, de propanol ou d'isopropanol, on peut aussi citer les polyols comme le propylèneglycol, le dipropylène glycol, le butylèneglycol, l'hexylèneglycol, la glycérine ou l'octanediol-1,2.

Selon un aspect préféré de la présente invention la composition telle que décrite précédemment comprend de 15% à 60%, plus particulièrement, de 20% à 40% en poids d'au moins un composé de formule (I) et de 10% à 40% en poids et de préférence de 15% à 30% en poids d'au moins un composé de formule (II). L'invention a aussi pour objet, utilisation de la composition telle que définie précédemment, pour préparer des compositions cosmétiques, dermocosmétiques, dermopharmaceutiques, pharmaceutiques. Les compositions cosmétiques, dermocosmétiques, dermopharmaceutiques ou pharmaceutiques ainsi préparées, contiennent généralement de 0,1% à 10% en poids et plus particulièrement de 1% à 3% en poids de la composition telle que définie précédemment. Les compositions cosmétiques, cosmétotextiles, dermocosmétiques, dermopharmaceutiques ou pharmaceutiques sont notamment à action protectrice, nourrissante, anti-stress, gainante et restructurante. Elles sont plus particulièrement destinées au traitement du cuir chevelu, des cheveux ou des follicules pileux.

L'invention a aussi pour objet, l'utilisation de la composition telle que définie précédemment, pour préparer des compositions destinées à être déposées, absorbées ou imprégnées sur, ou par des supports tissés ou non tissés, tels que par exemple un vêtement ou sous- vêtement, afin que celui-ci procure une sensation de bien-être à celui qui le porte.

Grâce à ses propriétés apaisantes, la composition selon l'invention peut être utilisée dans tous les produits contenant des composants plus ou moins irritants, de façon à améliorer leur tolérance comme, par exemple, dans les produits antipelliculaires. La composition selon l'invention peut également être utilisée en synergie avec d'autres produits habituellement utilisés pour préparer les produits topiques. Il s'agit notamment des produits apaisants comme l'alpha-bisabolol, les dérivés de réglisse, tels que l'acide glycyrrhétinique ou ses dérivés ou l'allantoine, des huiles hyperoxygénées comme l'épaline, des huiles, des cires essentielles, des produits à base d'oligosaccharides, des produits à base de peptides, des extraits de plantes comme par exemple, l'Aloe Vera ou Centella asiatica, des extraits d'algues, des sels minéraux (ou des produits à base de minéraux), des produits connus pour leur propriété antiradicalaire comme les polyphénols, le glutathion, les vitamines telles que la vitamine E ou vitamine C, les enzymes tels que la superoxyde dismutase ou la glutathion peroxydase, des produits connus pour leur propriété anti-inflammatoire, des vitamines en général (ou des produits à base de vitamines), des enzymes en général (ou des produits à base d'enzymes), des produits ayant une activité vis à vis de neuromédiateurs.

Selon l'utilisation prévue, la composition telle que décrite précédemment, est mise en oeuvre à des concentrations différentes et dans une formulation appropriée à cette utilisation. De telles compositions cosmétiques, dermocosmétiques, dermopharmaceutiques ou pharmaceutiques se présentent habituellement sous forme de solutions aqueuses, de solutions alcooliques diluées, d'huiles ou d'émulsions simples ou multiples, telles que les émulsions eau dans huile (E/H), huile dans eau (H/E) ou eau dans huile dans eau (E/H/E), dans lesquelles les huiles sont de nature végétale minérale ou synthétiques comme par exemple, les huiles de silicone. Comme formulation cosmétique, on peut citer, les huiles, comme par exemple les huiles capillaires, les crèmes, les gels, les laits, les lotions, les gels douches, les gels-crème, les savons, les savons liquides, les syndets ou les shampooings.

De telles formulations sont connues de l'homme du métier; leur préparation sont décrites, par exemple, dans les demandes de brevet publiées sous les numéros, WO92/06778, WO93128204. WO95/13863, WO95/35089 ou WO96/22109.

L'invention a donc aussi pour objet, une formulation cosmétique susceptible d'être obtenue par dilution du 1/10 jusqu'au 1/20000, de préférence du 1/10 au 1/100, de la composition telle que décrite précédemment, dans un ou plusieurs excipients cosmétiquement acceptables, et notamment une formulation cosmétique sous forme d'une émulsion huile dans eau ayant l'aspect d'un lait ayant une viscosité inférieure à 1Pa.s. comprenant comme émulsionnant une composition auto-émulsionnable à base d'alcools gras.

Comme compositions émulsionnantes préférées, on peut citer les MONTANOV™ 68, MONTANOV™ 14, MONTANOV™ 82 et MONTANOV™ 202 ou MONTANOV™ WO18, commercialisés par la société SEPPIC.

Selon le caractère que l'on peut donner à la formulation cosmétique, on peut le cas échéant, ajouter un latex inverse tel que le SEPIGEL™ 305, le SEPIGEL™ 501, le SIMULGEL™ 600 ou le SIMULGEL™ EG. Le terme dilution employé dans ce qui précède, englobe dans son acception la plus large, toutes les étapes permettant de passer de la composition telle que définie précédemment à la formulation cosmétique destinée à être commercialisée. Dans un autre mode préféré de la présente invention, la formulation cosmétique est une crème ou un lait apaisant pour traiter le cuir chevelu. Dans un autre mode préféré de la présente invention, la formulation cosmétique est une formule moussante ou un shampooing.

Les exemples suivants illustrent l'invention sans toutefois la limiter.

### Exemple 1 : Préparation d'une composition selon l'invention et mise en évidence de ses propriétés.

### A) Préparation

On prépare une composition (A) selon l'invention, par mélange sous agitation, de 75 grammes de PROTEOL™ SAV 50, qui est un mélange à environ entre 30% et 40% en poids de matière active, de N-cocoyl aminoacides avec 25 grammes de PECOSIL™ SPP 50, constitué à 100% de potassium diméthicone copolyol panthényl phosphate.

### B) Mise en évidence des propriétés inhibitrices de l'élastase leucocitaire humaine de la composition selon l'invention

### a) Principe du test

L'élastase leucocytaire humaine (ELH) intervient dans un grand nombre de pathologies inflammatoires. Cet enzyme est en particulier capable de dégrader de nombreuses macromolécules comme l'élastine fibreuse, certains types de collagène, les protéoglycanes, les glycoprotéines. Pour cette raison, l'ELH constitue un des maillons de la chaîne des réactions accompagnant le phénomène d'inflammation. Le blocage de cet enzyme par un effet "anti-élastase", permet donc d'empêcher la dégradation des molécules précitées et donc d'inhiber le processus de l'inflammation. Les propriétés "anti-élastase" d'un produit donné, peuvent être mises en évidence par un test in-vitro, à l'aide d'une substance qui est dégradée par l'ELH en se colorant, dans lequel les variations de coloration sont déterminées par spectrophotométrie. La substance utilisée dans le présent test est le N-méthoxysuccinyl-alanine-proline-valine-para-nitroanilide, substance normalement incolore qui libère, par hydrolyse par l'ELH, le para-nitroanilide, dont la cinétique d'apparition est suivie par spectrophotométrie à 410 nm. La réaction est réalisée dans un spectrophotomètre thermostaté à 25°C, disposant d'un passeur d'échantillons. Toutes les cinétiques sont réalisées au minimum trois fois, la moyenne et l'écart-type étant alors calculés pour les trois valeurs obtenues. La présence d'une molécule à activité "anti-élastase", se traduit par une limitation de l'apparition du produit coloré et cet effet est calculé par rapport à une courbe témoin obtenue en l'absence de ladite molécule. Il existe ainsi une corrélation entre le pourcentage d'inhibition de l'apparition du produit coloré par le composé testé et le pourcentage d'inhibition de l'ELH. Le pourcentage d'inhibition ainsi calculé est également représentatif de l'activité apaisante du composé testé.

### b) Essai

On prépare les solutions aqueuses suivantes :
- Solution 1 : solution aqueuse à 2,5% en matière active, de PECOSIL™ SPP 50 ;
- Solution 2 : solution aqueuse à 0,015% en matière active, de PROTEOL™ SAV 50S ;
- Solution 3 : solution aqueuse à 2,5% en matière active, de PECOSIL™ SPP 50 et à 0,015% en matière active, de PROTEOL™ SAV 50S.

On mesure le pourcentage d'inhibition de l'apparition du para-nitro anilide, pour chacune des ces trois solutions à l'aide d'un spectrophotomètre à 410 nm selon le protocole décrit au paragraphe précédent.

Les résultats sont consignés dans le tableau suivant :

| | % inhibition |
|---|---|
| solution 1 | 30% |
| solution 2 | 36% |
| solution 3 | 89% |

Ces résultats montrent, qu'à des concentrations pour lesquelles le PROTEOL™ SAV 50S (solution 2) et le PECOSIL™ SPP 50 (solution 1), ont une activité limitée (36% et 30%), la combinaison des deux produits présente de façon inattendue une activité supérieure (89% d'inhibition).

### C) Mise en évidence des propriétés inhibitrices de formation des peroxydes de la composition selon l'invention.

### a) Principe de l'étude "Ex Vivo"

L'effet protecteur de la composition (A), est évalué en déterminant le taux de peroxydes présents à la surface de mèches de cheveux soumis à une irradiation ultra violette de type UVA à une puissance de 25 Joules / cm², la composition (A) ayant été appliquée avant irradiation ou après irradiation. Le dosage des peroxydes lipidiques est effectué par analyse de la fluorescence induite par l'oxydation de la dichlorofluorescéine qu'ils provoquent. Le taux de peroxydes présents à la surface des cheveux est exprimé en unités UF par mg de cheveux.

### b) Essai

Cette étude est réalisée avec 40 mèches de cheveux prélevées sur des volontaires sains, préalablement délipidées dans de l'éthanol à 70°, puis rincées. Elles sont réparties en quatre lots de 10 mèches dénommés lots A, B, C et D. Les lots de mèches subissent les traitements suivants :
- Les mèches du lot A sont trempées dans de l'eau pendant 10 minutes puis séchées à l'air libre pendant 30 minutes.
- Les mèches du lot B sont trempées dans de l'eau pendant 10 minutes, séchées à l'air libre pendant 30 minutes puis placées dans un dispositif d'irradiation CILBER LOURMAT™ et soumises à une irradiation UVA de 25 Joules/cm².
- Les mèches du lot C sont trempées dans une solution aqueuse à 1% en poids de composition (A) pendant 10 minutes, séchées à l'air libre pendant 30 minutes puis placées dans un dispositif d'irradiation CILBER LOURMAT™ et soumises à une irradiation UVA de 25 Joules/cm².
- Les mèches du lot D sont trempées dans de l'eaù pendant 10 minutes, séchées à l'air libre pendant 30 minutes, placées dans un dispositif d'irradiation VILBER LOURMAT™ et soumises à une irradiation UVA de 25 Joules/cm² puis elles sont trempées dans une solution aqueuse à 1% en poids de composition (A) pendant 10 minutes et séchées à l'air libre pendant 30 minutes.

Le dosage des peroxydes est réalisé 24 heures après l'irradiation. L'ensemble des mèches est rincé puis pesé et mis en contact avec la dichlorofluorescéine. La fluorescence est mesurée au Fluoroskan™ Elle est ensuite pondérée par rapport à la masse de cheveux.

Les résultats sont consignés dans tableau suivant :

| | Lot A | Lot B | Lot C | Lot D |
|---|---|---|---|---|
| Taux de peroxydes lipidiques (en UF/mg de cheveux) | 354 | 672 | 535 | 447 |
| Taux relatif (base 100 = lot B) | 52.7 | 100 | 79,6 | 66,5 |

Ces résultats mettent en évidence les actions protectrices et réparatrices de la composition (A), vis à vis de l'action des rayons ultra violets de type UVA.

### D) Mise en évidence des propriétés anti-radicataires de la composition selon l'invention.

### a) Principe de l'étude

La détermination de l'effet antiradicalaire est fondée sur la capacité qu'a la molécule à étudier, d'inhiber ou de diminuer la vitesse de réduction du cytochrome C, lorsqu'elle est ajoutée au milieu réactionnel. L'anion superoxyde est formé par l'action de la xanthine oxydase sur la xanthine. Il induit, en l'absence d'une molécule capable de le capter, la réduction du cytochrome C. L'apparition de cytochrome C réduit, est suivie au spectrophotomètre à 550 nm, en présence (Essai) et en absence (Témoin), de molécules antiradicalaires.

### b) Essai

L'étude consiste à comparer l'activité antiradicalaire de la composition (A) avec celle de la vitamine C (acide ascorbique) et celle du panthénol ou D(+)-2,4-dihydroxy N-(3-hydroxy propyl) 3,3-diméthyl butanamide, qui est un composé couramment utilisé pour protéger les cheveux.

La réaction est effectuée dans un spectrophotomètre thermostaté à 25°C et disposant d'un passeur d'échantillons. Toutes les cinétiques sont déterminées au minimum trois fois ; la moyenne et l'écart type sont calculés pour les trois valeurs obtenues. Un pourcentage d'inhibition de la vitesse d'apparition du produit coloré (correspondant à la quantité d'anion superoxyde libre) est donc calculé pour chaque actif testé. Le calcul est effectué par rapport à la vitesse d'apparition du produit coloré dans le témoin (sans actif). Le pourcentage d'inhibition de rapparition du produit coloré par l'actif, correspond donc au pourcentage d'inhibition de l'anion superoxyde. Les résultats sont consignés dans tableau suivant :

| Produits | Concentrations testées | % d'inhibition antiradicalaire |
|---|---|---|
| Vitamine C | 0,46% (en matière active) | 94% |
| panthénol | 0,46% (en matière active) | 0% |
| Composition (A) | 0,46% (en matière active) | 94%. |

Ces résultats mettent en évidence une activité antiradicalaire de la composition (A) du même ordre que celle de la vitamine C, contrairement à celle inexistante du panthénol.

### E) Mise en évidence de l'effet stimulateur de la division cellulaire, de la composition selon l'invention.

### a) Principe de l'étude "in vitro"

L'effet des actifs sur la division cellulaire est mesuré par un dosage fluorimétrique du contenu en ADN de kératinocytes humains normaux soumis à un stress thermique élevé (20 minutes à 50°C) La quantité d'ADN présente dans les cellules, détermine leur capacité à se diviser. Les cellules sont utilisées à 60% de confluence. Elles sont incubées pendant 24 heures en présence des actifs. La division cellulaire est déterminée par dosage de la quantité d'ADN présente par puits.

### b) Essai

Quatre échantillons A, B, C et D, d'une culture de kératinocytes humains normaux, subissent les traitements respectifs suivants :
- Echantillon A : cellules non traitées et laissées à température ambiante.
- Echantillon B : cellules non traitées et chauffées à 50°C pendant 20 minutes.
- Echantillon C : cellules laissées incubées pendant 24 heures avec la composition (A) (concentration : 4,75 10⁻⁶ % en poids de matière active) et chauffées à 50°C pendant 20 minutes.
- Echantillon D : cellules laissées incubées pendant 24 heures avec le panthénol (concentration : 12,5 10⁻⁶ % en poids de matière active) et chauffées à 50°C pendant 20 minutes.

On effectue ensuite le dosage d'ADN de chacun des échantillons.

Les résultats, exprimés en *µ*g/puits, sont consignés dans le tableau suivant:

| | Echantillon A | Echantillon B | Echantilton C | Echantillon D |
|---|---|---|---|---|
| division cellulaire (en *µ*g/puits) | 4,5 | 3,7 | 5,7 | 4,3 |
| Taux relatif (Base 100 : Lot B) | 116,2 | 100 | 154,1 | 116,2 |

Ces résultats mettent en évidence la capacité de la composition (A), à stimuler la division cellulaire de façon à surpasser les effets néfastes de la chaleur, alors que le panthénol compense seulement ces effets.

### F) Mise en évidence de l'effet protecteur du métabolisme protéique de la racine du cheveu, de la composition selon l'invention.

### a) Principe de l'étude "in vitro"

L'effet de la protection du métabolisme des protéines, est déterminé par dosage colorimétrique du contenu en protéines des cellules (exprimé en µg/ml), après incubation pendant 24 heures en présence des actifs. Les cellules sont utilisées à 60% de confluence.

### b) Essai

Cinq échantillons E, F, G, H et I, d'une culture de kératinocytes humains normaux sont laissés incubés 24 heures en présence ou non d'une des solutions 1, 2 ou 3 suivantes :
- Solution 1: solution aqueuse à 1,25 10⁻⁵% en matière active, de PECOSIL™ SPP 50 ;
- Solution 2 : solution aqueuse à 1,125 10⁻⁵% en matière active, de PROTEOL™ SAV 50S ;
- Solution 3 : solution aqueuse à 1,25 10⁻⁵% en matière active, de PECOSIL™SPP 50 et à 1,125 10⁻⁵% en matière active, de PROTEOL™SAV 50S.
Ils sont ensuite soumis ou non à une température de 50°C pendant 20 minutes :
- Echantillon E : cellules non traitées et laissées à température ambiante.
- Echantillon F : cellules non traitées et chauffées à 50°C.
- Echantillon G : cellules traitées avec la solution 1 et chauffées à 50°C.
- Echantillon H : cellules traitées avec la solution 2 et chauffées à 50°C.
- Echantillon I : cellules traitées avec la solution 3 et chauffées à 50°C.
On effectue enfin le dosage d'ADN de chacun des échantillons.

Les résultats, exprimés en µg/ml, sont consignés dans le tableau suivant :

| Echantillon | E | F | G | H | 1 |
|---|---|---|---|---|---|
| Taux de protéines cellulaires en *µ*g/ml | 7,7 | 5,6 | 5,7 | 8,2 | 10,5 |
| Taux relatif (Base 100 : Echantillon F) | 138 | 100 | 102 | 146 | 188 |

Ces résultats mettent en évidence la capacité de la composition (A), à stimuler le métabolisme protéique des kératinocytes pour s'opposer aux effets néfastes de la chaleur, alors que les composés de formules (II) seuls, sont inactifs et que les composées de formule (I) seuls, sont moyennement actifs.

### G) Mise en évidence de l'effet inhibiteur de la dégradation des protéines des cheveux, de la composition selon l'invention.

### a) Principe de l'étude "Ex vivo"

L'effet protecteur et l'action préventives de la composition (A), sont évalués en mesurant la fluorescence intrinsèque du tryptophane de la kératine des mèches stressées soit par des rayons ultra-violets, soit par la chaleur. La dégradation protéique est caractérisée par une diminution de la fluorescence naturelle intrinsèque du tryptophane.

### b) Essai

Cette étude est réalisée avec 40 mèches de cheveux prélevées sur des volontaires sains, préalablement délipidées dans de l'éthanol à 70°, puis rincées. Elles sont réparties en cinq lots de 10 mèches, dénommés lots J, K, L, M et N, qui sont traités comme suit :.
- Les mèches du lot J sont trempées dans de l'eau pendant 10 minutes, séchées à l'air libre pendant 30 minutes (lot non traité ne subissant aucun stress).
- Les mèches du lot K sont trempées dans de l'eau pendant 10 minutes, séchées à l'air libre pendant 30 minutes puis placées dans un dispositif d'irradiation VILBER LOURMAT™ et soumises à une irradiation UVA de 25 Joules/cm².
- Les mèches du lot L sont trempées dans de l'eau pendant 10 minutes, séchées à l'air libre pendant 30 minutes puis elles sont soumises à un flux d'air à 90°C pendant 1 heure puis elles sont laissées à température ambiante pendant 15 minutes.
- Les mèches du lot M sont trempées dans une solution aqueuse à 1% en poids de composition (A) pendant 10 minutes, séchées à l'air libre pendant 30 minutes puis placées dans un dispositif d'irradiation VILBER LOURMAT™ et soumises à une irradiation UVA de 25 Joules/cm².
- Les mèches du lot N sont trempées dans une solution aqueuse à 1% en poids de composition (A) pendant 10 minutes, séchées à l'air libre pendant 30 minutes puis elles sont soumises à un flux d'air à 90°C pendant 1 heure puis elles sont laissées à température ambiante pendant 15 minutes.

L'intensité de la fluorescence est mesurée au moyen d'un spectrophotomètre CD60 DESAGA™. L'acquisition de la fluorescence est réalisée sur une surface de cheveu fixe et déterminée (350 mm²) et dans le sens longitudinal (équivalent au sens de la racine vers la pointe). La valeur d'intensité de fluorescence est déterminée en unités arbitraires ramenées à la masse du cheveu.

Les résultats exprimés en dégradation de fluorescence intrinsèque du tryptophane par gramme de cheveux, par rapport au lot J non traité et non stressé (Δ = 0), sont consignés dans le tableau suivant :

| | Lot J | Lot K | Lot L | Lot M | Lot N |
|---|---|---|---|---|---|
| Δ (en UA/g) | 0 | -193 | -163 | -73 | -107 |
| Taux relatif de dégradation (Base 100 : Lot K) | - | 100 | - | 38 | - |
| Taux relatif de dégradation (Base 100 : Lot L) | - | - | 100 | - | 66 |

Ces résultats mettent en évidence la capacité de la composition A, à ralentir la dégradation de la kératine des cheveux subissant un stress thermique ou un stress photochimique.

### H) Mise en évidence de l'effet inhibiteur de la formation d'écailles à la surface des cheveux, de la composition selon l'invention.

### a) Principe de l'étude "Ex Vivo"

La capacité d'une solution aqueuse à 3% en poids de composition (A), dénommée solution 4, à empêcher la formation des écailles sur la surface du cheveux a été testé sur des cheveux abîmés soumis à un stress thermique.

Cette propriété est mise en évidence par l'observation de la surface des cheveux traités et non traités par microscopie électronique à balayage.

### b) Essai

On prélève des cheveux de volontaires sains. 5 cheveux de chaque volontaire sont trempés pendant 10 minutes, soit dans la solution 4, soit dans l'eau (placébo). Tous les cheveux sont séchés à l'air libre puis ils sont soumis à un flux d'air à 90°C pendant 1 heure puis laissés à température ambiante pendant 15 minutes et observés par microscopie électronique à balayage et réalisation de clichés.

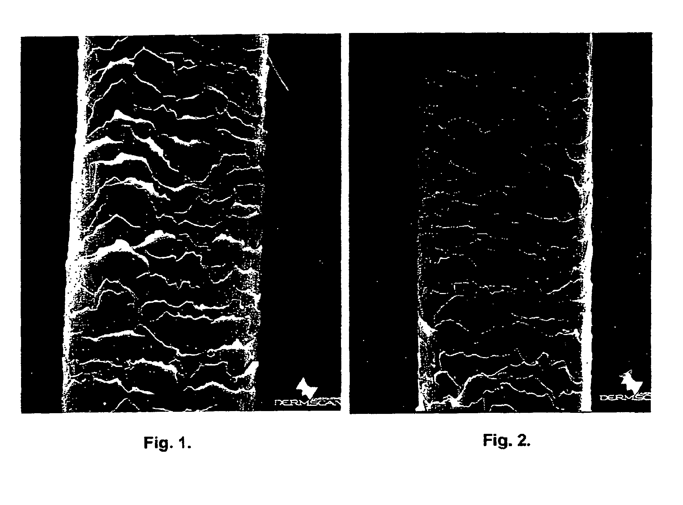

Les figures 1 et 2 mettent en évidence l'effet protecteur de la composition de l'invention, quant à la formation des écailles à la surface du cheveux.

Les exemples suivants illustrent la mise en oeuvre de la composition (A) pour préparer des formulations cosmétiques.

### Exemple 2 : Lotion capillaire thermoactive

| **Formule** | |
|---|---|
| Butylène glycol : | 3,0% |
| SIMULSOL™1293 : | 3.0% |
| Composition (A) : | 1,0% |
| Acide lactique : | QS pH = 6 |
| SEPICIDE™ HB: | 0,2% |
| SEPICIDE™CI : | 0,3% |
| Parfum : | 0,3% |
| Eau : | QSP 100% |

### Exemple 3 : Shampooing protecteur et relaxant

| **Formule** | |
|---|---|
| Amonyl™ 675 SB : | 5.0% |
| Sodium lauryl éther sulfate à 28% : | 35,0% |
| Composition (A) : | 1,0% |
| Capigel™ 98 : | 3.0% |
| SEPICIDE™ HB: | 0.5% |
| SEPICIDE™CI: | 0,3% |
| Soude : | QS pH =7,2 |
| Parfum: | 0.3% |
| Colorant (FDC bleu 1/jaune 5) : | QS |
| Eau : | QSP 100% |

### Caractéristiques

Le shampooing obtenu a un aspect limpide vert. Son pH est environ égal à 7.2 et sa viscosité est égale à 1000 cps (BROOKFIELD™ LVT : M4 V6).

### Exemple 4 : Protecteur "leave-on" ;Soin anti-stress pour cheveux

| **Formule** | |
|---|---|
| KETROL™ T: | 0,5% |
| Composition (A) : | 3,0% |
| Butylèneglycol: | 5,0% |
| DC 1501: | 5,0% |
| SIMULGEL™ EG : | 4,0% |
| SEPICIDE™ HB: | 0,5% |
| SEPICIDE™ Cl: | 0,3% |
| Parfum : | 0,3% |
| Eau: | QSP 100% |

### Caractéristiques

Le soin obtenu est sous forme de gel opaque. Son pH est environ égal à 6,5 et sa viscosité est égale à 40 000 cps (BROOKFIELD™ LVT : M4 V6).

### Exemple 5 : Masque crème "rince off" restructurant pour cheveux stressés et fragilisés

| **Formule** | |
|---|---|
| KETROL™T: | 0.5% |
| Composition (A) : | 3,0% |
| Butylèneglycol: | 3,0% |
| SIMULGEL™EG: | 1,0% |
| MONTANOV™82: | 3.0% |
| Huile de jojoba: | 1,0% |
| LANOL™P: | 6,0% |
| AMONYL™DM: | 1.0% |
| LANOL™99: | 5,0% |
| SIMULGEL™ EG : | 4.0% |
| SEPICIDE™ HB: | 0,3% |
| SEPICIDE™ Cl: | 0,2% |
| Parfum: | 0,2% |
| Eau: | QSP 100% |

### Caractéristiques

Le masque obtenu est sous forme de crème. Son pH est environ égal à 6,2 et sa viscosité est égale à 40 000 cps (BROOKFIELD™ LVT : M4 V6).

Les définitions des produits commerciaux utilisés dans les exemples sont les suivantes :
SIMULSOL™ 1293 est de l'huile de castor hydrogénée et éthoxylée, avec un indice d'éthoxylation égal à 40, commercialisé par la société SEPPIC.
SEPICIDE™ HB est un mélange conservateur comprenant du Phenoxyethanol, du méthylparaben, de l'éthylparaben, du propylparaben et du butyl paraben, commercialisé par la société SEPPIC.
SEPICIDE™ Cl est de l'imidazolidinyl urée, commercialisé par la société SEPPIC.
CAPIGEL™ 98 est un épaississant liquide à base de copolymère acrylate commercialisé pas la société SEPPIC.
AMONYL™ 675SB est une sulfobétaïne commercialisée par la société SEPPIC.
SIMULGEL™EG est un latex inverse de copolymère (dénomination INCI : Sodium acrylate/Sodium acryloyldimethyltaurate copolymer and Isohexadecane and Polysorbate 80) commercialisé par la société SEPPIC.
KETROL™ T est de la gomme de xanthane commercialisée par la société KELCO. LANOL™99 est de l'isononyl isononanoate commercialisé par la société SEPPIC.
DC1501 est un mélange de cyclopentasiloxane et de diméthiconol commercialisé par la société DOW CHEMICAL.
MONTANQV™82 est un agent émulsionnant à base d'alcool cétèarylique et de co-coylglucoside.

## Revendications

1. Composition **caractérisée en ce qu'**elle comprend à titre de principe actif, au moins un composé de formule (I): 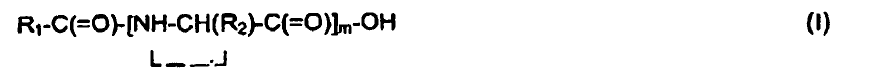 ou ses sels, dans laquelle R₁ représente la chaîne caractérisante d'un acide gras, saturé ou insaturé, linéaire ou ramifié, comportant de 8 à 30 atomes de carbone, R₂ représente la chaîne caractérisante d'un acide aminé et m est compris entre 1 et 5, et au moins un composé de formule (II):
ZO-(CH₂)₃-NH-C(=O)-CH(OH)-C(CH₃)₂-CH₂,-OH (II)
dans laquelle Z. représente un radical dérivé du phosphore de formule:
P(R₃O)(OM)(=O)-,
dans lequel OM représente un radical OH libre ou salifié, et R₃ un radical dérivé de polysiloxanes alkoxylés

2. Composition telle que définie à la revendication 1, pour laquelle, dans la formule (I), le groupe R₁-C(=O)- comporte de 8 à 22 atomes de carbone et plus particulièrement un des radicaux octanoyle (caprylyle), décanoyle, undécylènoyle, dodécanoyle (lauroyle), tétradécanoyle (myristyle), hexadécanoyle (palmitoyle), octadécanoyle (stéaryle), eicosanoyle (arachidoyle), docosanoyle (béhènoyle), 8-octadécènoyle (oléyle), éicosènoyle (gadoloyle), 13-docosènoyle (érucyle), 9,12-octadécadiènoyle (linoléoyle) ou 9,12,15-octadécatriénoyle (linolénoyle).

3. Composition telle que définie à la revendication 2, pour laquelle, dans la formule (I), le fragment R₁-CO (I) comporte de 12 à 18 atomes de carbone.

4. Composition telle que définie à l'une quelconques des revendications 1 à 3, comportant au moins un composé de formule (I) choisi parmi les dérivés N-acylés de l'acide glutamique, de l'acide aspartique, de l'alanine ou de la glycine.

5. Composition telle que définie à l'une des revendications 1 à 4, pour laquelle, dans la formule (I), m est un nombre décimal inférieur ou égal à 2, plus particulièrement inférieur ou égal à 1,4.

6. Composition telle que définie à l'une des revendications 1 à 4, pour laquelle, dans la formule (I), m est égal à 1.

7. Composition telle que définie à l'une des revendications 1 à 6, comprenant un seul composé de la formule (I).

8. Composition telle que définie à l'une des revendications 1 à 6, comprenant un mélange de composés de la formules (I) et notamment, ou bien un mélange de composés de formules (I) comportant toutes le même fragment R₁-CO (I), ou bien un mélange de composés de formules (I) comportant toutes le même fragment :

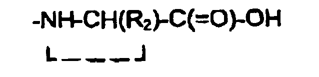

9. Composition telle que définie à la revendication 8, comprenant un mélange de composés de la formules (I) comportant toutes le même fragment R₁-CO (I).

10. Composition telle que définie à la revendication 8, comprenant un mélange de composés de la formules (I) comportant toutes le même fragment :

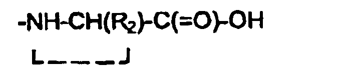

11. Composition telle que définie à l'une quelconque des revendications 1 à 9, pour laquelle le composé de formule (I) est un N-lauroyl aminoacide ou un mélange de N-cocoyl aminoacides.

12. Composition telle que définie à l'une quelconque des revendications 1 à 11, dans laquelle le composé de formule (II) est le potassium diméthicone copolyol panthényl phosphate.

13. Composition telle que définie à la revendication 12 **caractérisée en ce qu'**elle comprend outre un polysiloxane alkoxylé et phosphaté plus particulièrement un sel de diméthicone copolyol phosphate.

14. Composition telle que décrite à l'une des revendications 1 à 13., **caractérisée en ce qu'**elle comprend un ou plusieurs véhicules minéraux ou organiques choisis parmi, l'eau, les solutions aqueuses d'éthanol, de propanol ou d'isopropanol, le propylèneglycol, le dipropylène glycol, le butylèneglycol, l'hexylèneglycol, la glycérine ou l'octanediol-1,2.

15. Composition telle que décrite à l'une des revendications 1à 14, **caractérisée en ce qu'**elle comprend de 15% à 60%, plus particulièrement, de 20% à 40% en poids d'au moins un composé de formule (I) et de 10% à 40% en poids et de préférence de 15% à 30% en poids d'au moins un composé de formule (II).

16. Utilisation de la composition telle que définie à rune des revendications 1 à 15, pour préparer des compositions cosmétiques, dermocosmétiques, dermopharmaceutiques, pharmaceutiques.

17. Utilisation de la composition telle que définie à l'une des revendications 1 à 15, pour préparer des compositions destinées à être déposées, absorbées ou imprégnées sur, ou par des supports tissés ou non tissés, tels que par exemple un vêtement ou sous- vêtement

18. Composition cosmétique, dermocosmétique, dermopharmaceutique ou pharmaceutique **caractérisée en ce qu'**elle comprend de 0,1% à 10% en poids et plus particulièrement de 1% à 3% en poids de la composition telle que définie à l'une des revendications 1 à 15.

19. Composition telle que définie à la revendication 18, à action protectrice, nourrissante, anti-stress, gainante et restructurante et plus particulièrement destinée au traitement du cuir chevelu, des cheveux ou des follicules pileux.

## Patentansprüche

1. Zusammensetzung, **dadurch gekennzeichnet, daß** sie als Wirkprinzip mindestens eine Zusammensetzung der Formel (I) 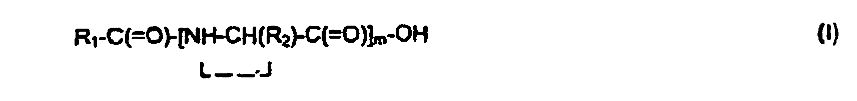 oder ihre Salze, wobei R₁ die charakterisierende Kette einer gesättigten oder ungesättigten geradkettigen oder verzweigten Fettsäure mit 8 bis 30 Kohlenstoffatomen bedeutet, R₂ die charakterisierende Kette einer Aminosäure bedeutet und m 1 bis 5 bedeutet, sowie mindestens eine Verbindung der Formel (II)
**ZO-(CH**_{**2**}**)**_{**3**}**-NH-C(=O)-CH(OH)-C(CH**_{**3**}**)**_{**2**}**-CK**_{**2**}**-OH** (II)
in der Z einen von Phosphor abgeleiteten Rest der Formel:
P(R₃O)(OM)(=O)-,
bedeutet, in dem OM einen freien oder in Salzform vorliegenden OH-Rest und R₃ einen von Alkoxypolysiloxanen abgeleiteten Rest bedeutet, enthält.

2. Zusammensetzung nach Anspruch 1, bei der in Formel (I) die Gruppe R₁-C(=O)- 8 bis 22 Kohlenstoffatome umfaßt, insbesondere einen der Reste Octanoyl (Caprylyl), Decanoyl, Undecylenoyl, Dodecanoyl (Lauroyl), Tetradecanoyl (Myristyl), Hexadecanoyl (Palmitoyl), Octadecanoyl (Stearyl), Eicosanoyl (Arachidoyl), Docosanoyl (Behenoyl), 8-Octadecenoyl (Oleyl), Eicosenoyl (Gadoloyl), 13-Docosenoyl (Erucyl), 9,12-Octadecadienoyl (Linoleoyl) und 9, 12, 15-0ctadecatrienoyl (Linolenoyl).

3. Zusammensetzung nach Anspruch 2, bei der in Formel (I) das Fragment R₁-CO (I) 12 bis 18 Kohlenstoffatome enthält.

4. Zusammensetzung nach einem der Ansprüche 1 bis 3, die mindestens eine Verbindung der Formel (I) aus der Gruppe der N-Acylderivate der Glutaminsäure, der Asparaginsäure, des Alanins oder des Glycins enthält.

5. Zusammensetzung nach einem der Ansprüche 1 bis 4, bei der in Formel (I), m eine Dezimalzahl kleiner oder gleich 2, insbesondere kleiner oder gleich 1,4 ist.

6. Zusammensetzung nach einem der Ansprüche 1 bis 4, bei der in Formel (I) m gleich 1 ist.

7. Zusammensetzung nach einem der Ansprüche 1 bis 6, die eine einzige Verbindung der Formel (I) enthält.

8. Zusammensetzung nach einem der Ansprüche 1 bis 6, die eine Mischung von Verbindungen der Formeln (I), und besonders oder auch eine Mischung von Verbindungen der Formeln (I), die alle das gleiche Fragment R₁-CO (I) enthalten, oder auch eine Mischung von Verbindungen der Formeln (I), die alle das gleiche Fragment 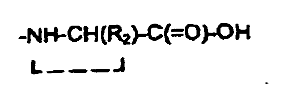 enthalten, enthält.

9. Zusammensetzung nach Anspruch 8, die eine Mischung von Verbindungen der Formeln (I), die alle das gleiche Fragment R₁-CO (I) enthalten, enthält.

10. Zusammensetzung nach Anspruch 8, die eine Mischung von Verbindungen der Formeln (I), die alle das gleiche Fragment 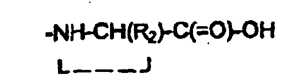 enthalten, enthält.

11. Zusammensetzung nach einem der Ansprüche 1 bis 9, bei der die Verbindung der Formel (I) eine N-Lauroylaminosäure oder eine Mischung aus N-Cocoylaminosäuren ist.

12. Zusammensetzung nach einem der Ansprüche 1 bis 11, bei der die Verbindung der Formel (II) Kaliumdimethiconcopolyolpanthenylphosphat ist.

13. Zusammensetzung nach Anspruch 12, **dadurch gekennzeichnet, daß** sie weiterhin ein phosphathaltiges Alkoxypolysiloxan, insbesondere ein Salz des Dimethiconcopolyolphosphats, enthält.

14. Zusammensetzung nach einem der Ansprüche 1 bis 13, **dadurch gekennzeichnet, daß** sie einen oder mehrere mineralische oder organische Träger aus der Gruppe Wasser, wäßrige Ethanol-, Propanol- oder Isopropanollösungen, Propylenglykol, Dipropylenglykol, Butylenglykol, Hexylenglykol, Glycerin oder Octan-1,2-diol enthält.

15. Zusammensetzung nach einem der Ansprüche 1 bis 14, **dadurch gekennzeichnet, daß** sie 15 Gew.-% bis 60 Gew.-%, insbesondere 20 Gew.-% bis 40 Gew.-%, an mindestens einer Verbindung der Formel (I) und 10 Gew.-% bis 40 Gew.-%, vorzugsweise 15 Gew.-% bis 30 Gew.-%, an mindestens einer Verbindung der Formel (II) enthält.

16. Verwendung der Zusammensetzung nach einem der Ansprüche 1 bis 15 zur Herstellung von kosmetischen, hautkosmetischen, hautpharmazeutischen bzw. pharmazeutischen Zusammensetzungen.

17. Verwendung der Zusammensetzung nach einem der Ansprüche 1 bis 15 zur Herstellung von Zusammensetzungen zur Aufbringung, Absorption oder Imprägnation auf oder durch gewebeartige oder vliesartige Träger, wie zum Beispiel Kleidung oder Unterkleidung.

18. Kosmetische, hautkosmetische, hautpharmazeutische oder pharmazeutische Zusammensetzung, **dadurch gekennzeichnet, daß** sie 0,1 Gew.-% bis 10 Gew.-%, insbesondere 1 Gew.-% bis 3 Gew.-%, der Zusammensetzung nach einem der Ansprüche 1 bis 15 enthält.

19. Zusammensetzung nach Anspruch 18 mit schützender, nährender, gegen Streß wirkender, umhüllender und restrukturierender Wirkung, insbesondere zur Behandlung der Kopfhaut, der Haare oder der Haarfollikel.

## Claims

1. Composition **characterized in that** it comprises as active principle at least one compound of formula (I): 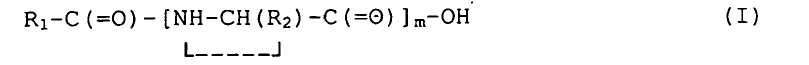 or salts thereof, in which R₁ represents the characterizing chain of a saturated or unsaturated, linear or branched fatty acid containing from 8 to 30 carbon atoms, R₂ represents the characterizing chain of an amino acid and m is between 1 and 5, and at least one compound of formula (II):
ZO- (CH₂)₃-NH-C (=O)-CH (OH)-C (CH₃)₂-CH₂-OH (II)
in which Z represents a phosphorus-based radical of formula:
P (R₃O) (OM) (=O)-,
in which OM represents a free or salified OH radical, and R₃ represents an alkoxylated polysiloxane-based radical.

2. Composition as defined in Claim 1, for which, in formula (I), the group R₁-C(=O)- contains from 8 to 22 carbon atoms and more particularly a radical from among octanoyl (caprylyl), decanoyl, undecylenoyl, dodecanoyl (lauroyl), tetradecanoyl (myristyl), hexadecanoyl (palmitoyl), octadecanoyl (stearyl), eicosanoyl (arachidoyl), docosanoyl (behenoyl), 8-octadecenoyl (oleyl), eicosenoyl (gadoloyl), 13-docosenoyl (erucyl), 9,12-octadecadienoyl (linoleoyl) and 9,12,15-octadecatrienoyl (linolenoyl).

3. Composition as defined in Claim 2, for which, in formula (I), the fragment R₁-CO (I) contains from 12 to 18 carbon atoms.

4. Composition as defined in any one of Claims 1 to 3, comprising at least one compound of formula (I) chosen from N-acyl derivatives of glutamic acid, of aspartic acid, of alanine or of glycine.

5. Composition as defined in one of Claims 1 to 4, for which, in formula (I), m is a decimal number less than or equal to 2 and more particularly less than or equal to 1.4.

6. Composition as defined in one of Claims 1 to 4, for which, in formula (I), m is equal to 1.

7. Composition as defined in one of Claims 1 to 6, comprising only one compound of formula (I).

8. Composition as defined in one of Claims 1 to 6, comprising a mixture of compounds of formula (I) and especially either a mixture of compounds of formula (I) all comprising the same fragment R₁-CO (I), or a mixture of compounds of formula (I) all comprising the same fragment:

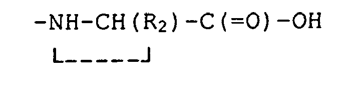

9. Composition as defined in Claim 8, comprising a mixture of compounds of formula (I) all comprising the same fragment R₁-CO (I).

10. Composition as defined in Claim 8, comprising a mixture of compounds of formula (I) all comprising the same fragment:

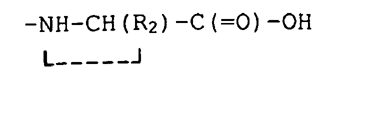

11. Composition as defined in any one of Claims 1 to 9, for which the compound of formula (I) is an N-lauroylamino acid or a mixture of N-cocoylamino acids.

12. Composition as defined in any one of Claims 1 to 11, in which the compound of formula (II) is potassium dimethicone copolyol panthenyl phosphate.

13. Composition as defined in Claim 12, **characterized in that** it also comprises an alkoxylated polysiloxane, phosphate, more particularly a dimethicone copolyol phosphate salt.

14. Composition as described in one of Claims 1 to 13, **characterized in that** it comprises one or more mineral or organic vehicles chosen from water, aqueous solutions of ethanol, propanol or isopropanol, propylene glycol, dipropylene glycol, butylene glycol, hexylene glycol, glycerol and 1,2-octanediol.

15. Composition as described in one of Claims 1 to 14, **characterized in that** it comprises from 15% to 60% and more particularly from 20% to 40% by weight of at least one compound of formula (I) and from 10% to 40% by weight and preferably from 15% to 30% by weight of at least one compound of formula (II).

16. Use of the composition as defined in one of Claims 1 to 15 for preparing cosmetic, dermocosmetic, dermopharmaceutical or pharmaceutical compositions.

17. Use of the composition as defined in one of Claims 1 to 15 for preparing compositions intended to be deposited, absorbed or impregnated onto or by woven or nonwoven supports, for instance a garment or undergarment.

18. Cosmetic, dermocosmetic, dermopharmaceutical or pharmaceutical composition, **characterized in that** it comprises from 0.1% to 10% by weight and more particularly from 1% to 3% by weight of the composition as defined in one of Claims 1 to 15.

19. Composition as defined in Claim 18, with protective, nourishing, stress-relieving, coating and restructuring action, more particularly intended for treating the scalp, the hair or hair follicles.
